# EUROPÄISCHE PATENTSCHRIFT

(11) **EP 1 635 897 B1**
(45) Veröffentlichungstag und Bekanntmachung des Hinweises auf die Patenterteilung: **18.07.2018**
(21) Anmeldenummer: 04739465.5
(22) Anmeldetag: 29.05.2004
(51) Int. Cl.: A61M 5/34, A61M 5/32

(54) **KAPPE FÜR INJEKTIONSVORRICHTUNGEN**
CAP FOR INJECTION DEVICES
CAPUCHON POUR DISPOSITIFS D'INJECTION

(30) Priorität: 13.06.2003 DE 10327119
(43) Veröffentlichungstag der Anmeldung: 22.03.2006
(73) Patentinhaber: Sanofi-Aventis Deutschland GmbH, 65929 Frankfurt am Main (DE)
(72) Erfinder: LARSEN, Carsten, 9520 Skorping (DK); POMMEREAU, Christian, 55278 Undenheim (DE)
(74) Vertreter: Keil & Schaafhausen Patent- und Rechtsanwälte PartGmbB
(86) Internationale Anmeldenummer: PCT/EP2004/005852
(87) Internationale Veröffentlichungsnummer: WO 2004/110534

(56) Entgegenhaltungen:
- WO-A-88/06463
- WO-A-90/03198
- DE-A1- 3 741 885
- US-A- 2 800 816
- US-A- 5 125 912
- US-A- 5 305 766
- US-A1- 2003 093 037

## Beschreibung

Die vorliegende Erfindung betrifft eine Kappe, die sich für eine austauschbare Nadelanordnung aufweisende Injektionsvorrichtung eignet und als Montage-/Demontagewerkzeug der Nadelanordnung dient.

Injektionsvorrichtungen mit austauschbaren Nadelanordnungen sind aus zahlreichen Patentveröffentlichungen, wie zum Beispiel der EP-A1-245312, EP-A1-268191, EP-A2-327910 ; EP-A1-594349, EP-A1-496141, EP-A1-450905, EP-A1-498737, WO 97/36625 A1, EP-A1-1003581 und WO2003011371 A1, wohlbekannt.

Weiterhin ist aus der DE 37 41 885 A1 ein Schreib- oder Zeichengerät bekannt, bei dem zum Ein- und Ausschrauben der Schreibspitze als Schraubwerkzeug die Kappe verwendet wird.

WO 90/03198 offenbart eine Vorrichtung mit einer Nadelvorrichtung, welche eine Nadel und einen Nadelträger aufweist, welcher koaxial zur Nadel ausgerichtet ist. Mittels einer Nadelpositioniervorrichtung kann die Nadel auf der Vorrichtung montiert werden und wieder von der Vorrichtung abgenommen werden. Die Nadelpositioniervorrichtung weist dazu Hülsen auf, die die Nadel koaxial umgeben können.

US 5,129,912 offenbart eine separate Vorrichtung zur Entfernung einer Nadel. Die Vorrichtung ist beidseitig offen und umgibt eine zu entfernende Nadel.

WO 88/06463 offenbart eine Vorrichtung mit einer Nadel und einem Nadelfutteral. Mithilfe des Nadelfutterals ist die Nadel von einer Injektionsvorrichtung entfernbar. Nadelfutteral und benutzte Nadel können zusammen miteinander entsorgt werden.

US 2003/0093037 offenbart eine Injektionsspritze mit einer Nadel und einer Nadelumhüllung. Die Nadelumhüllung kann mit der Nadel in Eingriff stehen, um die Nadel von der Injektionsspritze zu entfernen.

Einige dieser Injektionsvorrichtungen werden aufgrund ihrer im Wesentlichen länglichen Form und der Tatsache, dass die Vorrichtung eine das abzugebende Arzneimittel enthaltende Patrone enthält, als "Pen-Vorrichtungen" oder "Pens" bezeichnet. Es gibt Pens zum einmaligen Gebrauch und wiederverwendbare Pens. Im Allgemeinen umfassen beide Pen-Arten eine das Arzneimittel oder die pharmazeutische Zusammensetzung, das bzw. die zugeführt werden soll, enthaltende Patrone. Es ist ein besonderes Merkmal des wiederverwendbaren Pens, dass die Arzneimittelpatrone herausgenommen werden kann, wenn sie leer ist, und die Vorrichtung mit einer neuen wieder geladen werden kann. Bei dem Pen zum einmaligen Gebrauch ist hingegen kein Austausch der Patrone beabsichtigt, wobei der Pen fortgeworfen werden soll, wenn der Arzneimittelspeicher (meistens eine Patrone) leer ist.

Diese Art von Injektionsvorrichtungen eignet. sich für die subkutane/transdermale Verabreichung von pharmazeutischen Zusammensetzungen, zum Beispiel auf dem Gebiet der Diabetes (zum Beispiel Insuline, Insulinderivate), Behandlung oder Prophylaxe von thrombotischen Erkrankungen (zum Beispiel Heparine, Heparinderivate), Behandlung mittels Wachstumshormonen oder Impfung. Die Injektionsvorrichtungen sind zur Selbstverabreichung des Arzneimittels durch den Patienten ausgestaltet, wie dies zum Beispiel für Diabetiker erforderlich ist, oder zur Behandlung von anderen Erkrankungen, bei denen eine Langzeitbehandlung mit regelmäßigen Dosierungen eines subkutan verabreichten Arzneimittels erforderlich ist. Es können jedoch auch ähnliche oder identische Injektionsvorrichtungen bei der Tiergesundheit oder -impfung geeignet sein.

Im Allgemeinen umfasst diese Art von Injektionsvorrichtung ein aus einem oder mehreren Teilen bestehendes Gehäuse, einen Arzneimittelspeicher eines abzugebenden (flüssigen oder suspendierten) Arzneimittels, einen Dosier- und Arzneimittelzufuhrmechanismus (zum Beispiel Kolben, Antriebsmechanismus, Dosiermechanismus, Abgabemechanismus), der eine genaue Dosierung und Verabreichung einer vorgewählten Arzneimittelmenge gestattet, ein Injektionsnadelsystem und eine Kappe.

Die Arzneimittelspeicher der Vorrichtungen sind größtenteils gemäß der grundsätzlichen Funktion einer Patrone ausgestaltet, wobei das Arzneimittel durch einen bewegbaren Kolben ausgetrieben und dem Patienten durch eine Injektionsnadel verabreicht wird.

In einigen Fällen kann das Injektionsnadelsystem (die Nadelanordnung) weiterhin eine besondere "Nadelkappe" enthalten (die sich von der Kappe der Vorrichtung unterscheidet).

Der Fachmann kennt viele Möglichkeiten und Technologien der Ausführung oder Realisierung der besonderen Ausgestaltung oder des speziellen Aufbaus jedes der erforderlichen (gegebenenfalls mechanischen, elektrischen oder elektronisch ausgestatteten oder gestützten) Elemente einer solchen Injektionsvorrichtung (wie zum Beispiel ein Gehäuse, einen Arzneimittelspeicher (eine Arzneimittelpatrone), einen Antriebsmechanismus, einen Dosiermechanismus, einen Abgabemechanismus, eine Nadelanordnung, eine Kappe usw.)

Im Allgemeinen wird die dem Patienten zu verabreichende Arzneimitteldosis durch einen geeigneten Dosiseinstell- und Abgabemechanismus eingestellt und durch Verwendung einer Injektionsnadel (zum Beispiel Nadeln von 29 bis 33 Gauge) ausgetrieben. Für subkutane oder hypodermale Verabreichung von pharmazeutischen Zusammensetzungen geeignete Injektionsnadeln weisen eine Länge von 3-18 mm, meistens 5-13 mm auf.

Nach der Einrichtung der Nadelanordnung auf der Vorrichtung soll die Injektionsnadel regelmässig, vorzugsweise nach jeder Injektion, ausgetauscht werden, um die Sterilität des Systems zu sichern und Infektionen zu vermeiden. Demgemäss ist ein regelmässiger Austausch der Nadel für den Patienten äusserst wichtig.

Viele Patienten (zum Beispiel ältere Leute oder Diabetiker) sind eigentlich nicht in der Lage, die Nadel oder Nadelanordnung auszutauschen, wie dies aus den oben genannten Gründen erforderlich wäre.

Der Grund dafür kann irgendeine Behinderung bezüglich der Augen, Finger und dergleichen sein. Dies kann dann für solche Leute zu einer erhöhten Verletzungsgefahr durch Nadelstiche führen oder auch die Infektionsgefahr erhöhen, wenn die Nadel/Nadelanordnung nicht regelmässig ausgetauscht wird.

Hier reduziert die vorliegende Erfindung diese Gefahren und ermöglicht es der Patientengruppe, die Nadelanordnung ohne Hilfe sicher auszutauschen. Darüber hinaus, können auch andere Verwender der Erfindung, wie zum Beispiel Ärzte oder Krankenschwestern, die Vorteile der vorliegenden Erfindung nutzen.

Das oben genannte Problem wird durch die technischen Elemente der vorliegenden Erfindung gelöst. Demgemäß handelt es sich bei dem ersten Aspekt der vorliegenden Erfindung um eine Injektionsvorrichtung mit einer Kappe, wobei besagte Injektionsvorrichtung eine austauschbare Nadelanordnung umfasst und wobei die Kappe eine Befestigung zur Befestigung der Kappe an der Vorrichtung aufweist, um die austauschbare Nadelanordnung abzudecken, wobei die Kappe eine einseitige Öffnung aufweist und als Montage-/Demontagewerkzeug dient und wobei die Kappe eine Aufnahmebohrung umfasst, dadurch gekennzeichnet, dass die Kappe mit der Aufnahmebohrung zur Aufnahme der Nadelanordnung ausgestaltet ist.

Folglich stellt die vorliegende Erfindung ein Montage-/Demontagemittel/Werkzeug bereit, das sich für die oben genannten Patientengruppen besonders leicht verwenden lässt und an die besondere Geometrie der Nadelanordnung solcher Injektionsvorrichtungen angepasst ist.

Gemäß der vorliegenden Erfindung sollen die nachfolgend angeführten Begriffe neben dem allgemeinen Verständnis des Fachmanns die folgende, bevorzugte Bedeutung haben:
Eine "Injektionsvorrichtung" bedeutet eine beliebige Injektionsvorrichtung mit einer Kappe, zum Beispiel Pen-Injektionsvorrichtungen, die aus der EP-A1-828527, EP-A1-581924, EP-A1-498737, WO2003/011371, EP-A1-1074273 bekannt sind, oder andere Injektionsvorrichtungen mit einer Kappe, die zum Beispiel aus der WO2002/05147A1 oder WO2002/076535A1 bekannt sind.

Eine "austauschbare Nadelanordnung" bedeutet eine beliebige Nadelanordnung bzw. einen beliebigen Nadelhalter einer Injektionsvorrichtung mit einer Injektionsnadel, die zum Entfernen durch den Benutzer ausgestaltet ist (wie es zur Gewährleistung erforderlich wäre, dass von dem Benutzer oder Patienten eine saubere Nadel verwendet wird). In der Regel muss die besagte Nadelanordnung mit dem die Patrone enthaltenden Gehäuse verschraubt oder auf andere Weise daran befestigt werden. Deshalb kann die Nadelanordnung so ausgestaltet sein, dass sie sich leicht montieren/demontieren (oder verriegeln/entriegeln) lässt, indem sie zum Beispiel eine strukturierte Oberfläche aufweist oder wie eine Sechskant- oder MehrkantSchraube/Mutter ausgestaltet ist.

Die Nadel und/oder die Injektionsvorrichtung ist zur transdermalen oder subkutanen Verabreichung von pharmazeutischen Zusammensetzungen zur Anwendung auf Menschen oder Tiere ausgestaltet.

Besonders bevorzugt ist die Nadel und/oder die Injektionsvorrichtung zur Verabreichung von Insulin, Insulinderivaten oder dergleichen, Heparin, Heparinderivaten oder dergleichen, Wachstumshormonen und dergleichen ausgestaltet.

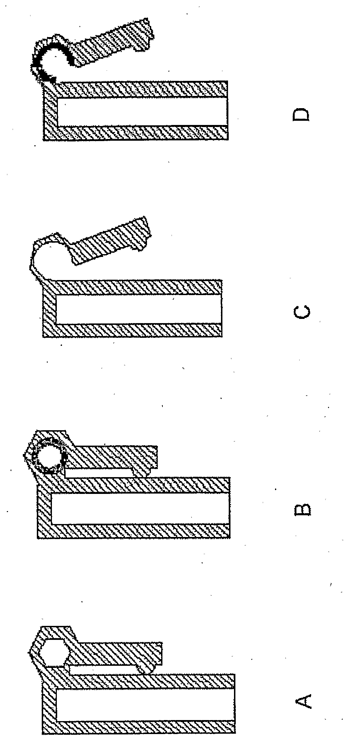

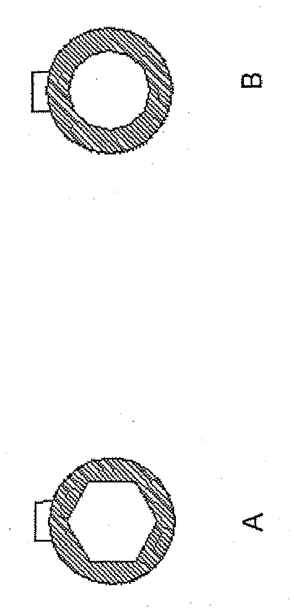

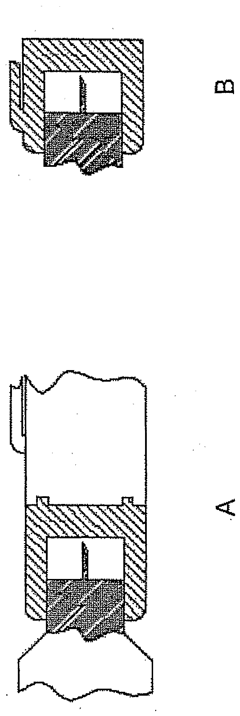

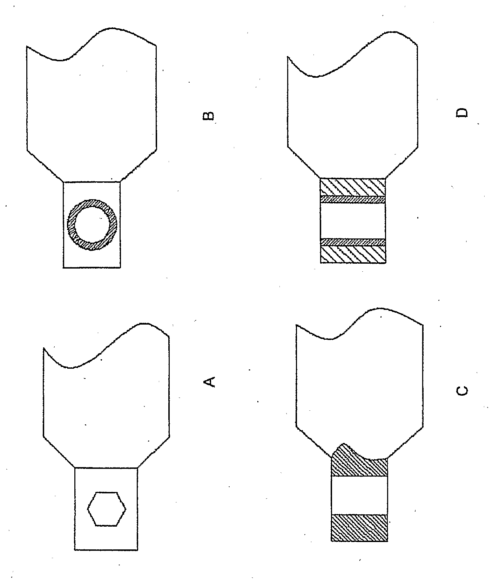

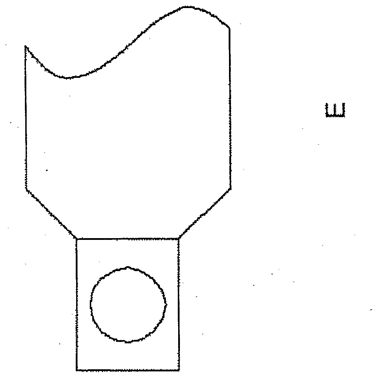

Bei einer "Kappe" gemäß der vorliegenden Erfindung kann es sich um eine beliebige geeignete Abdeckung von zumindest der Nadelanordnung einer Injektionsvorrichtung handeln, die gegebenenfalls eine Klemme und/oder Befestigung umfasst (siehe zum Beispiel Figuren 1 bis 4). Des weiteren kann es sich bei einer "Kappe" gemäß der vorliegenden Erfindung um die Originalkappe einer Injektionsvorrichtung oder einer Befestigung oder eines Anschlussstücks, die bzw. das an der Originalkappe der Injektionsvorrichtung befestigt sein kann und mit der Originalkappe form- und/oder kraftschlüssig verbunden ist, handeln.

Eine "Kappe, die an dem Vorrichtungsschaft befestigt werden kann" bedeutet eine beliebige Kappe, die in den oder aus dem Schaft einer Injektionsvorrichtung geschraubt, oder daran geklemmt oder auf andere Weise befestigt werden kann.

Eine "Aufnahmebohrung" der Kappe bedeutet eine beliebige Aufnahmebohrung, die mit der Nadelanordnung zusammenpasst, um eine Montage/Demontage der austauschbaren Nadelanordnung an der Injektionsvorrichtung zu gestatten. Die Aufnahmebohrung wird durch die Kappe, durch die optionale Klemme, durch eine beliebige Befestigung der Kappe oder durch eine Kombination der Kappe, Klemme oder Befestigung gebildet/ausgestaltet. Die Aufnahmebohrung ist erfindungsgemäß wie ein Schraubenschlüssel (Imbusschlüssel, Steckschlüssel, Ringschlüssel, Sechskant- oder Mehrkantschlüssel) oder Mutternschlüssel ausgestaltet, die auf die Größe des Durchmessers der Nadelanordnung einstellbar sind. Die Innenfläche der Aufnahmebohrung ist erfindungsgemäß mit Gummi bedeckt. Als Alternative dazu kann die Innenfläche der Aufnahmebohrung durch ein beliebiges Material bedeckt sein oder aus einem beliebigen Material bestehen, das zum Beispiel durch erhöhte Rauhigkeit der Oberfläche und/oder elastische Eigenschaften des verwendeten Materials einen geeigneten Reibungskoeffizienten aufweist (wie zum

Beispiel Gummi, geeignete Kunststoffe oder behandelte Oberflächen) und sich zur Montage/Demontage der Nadelanordnung der Injektionsvorrichtung eignet.

Bei einem zweiten Aspekt der vorliegenden Erfindung handelt es sich um eine Injektionsvorrichtung mit einer erfindungsgemäßen Kappe.

Bei einem dritten Aspekt der vorliegenden Erfindung handelt es sich um eine austauschbare Nadelanordnung mit der Geometrie einer Sechskant- oder Mehrkantschraube oder eines solchen Bolzens und die mit der Aufnahmebohrung der erfindungsgemäßen Kappe zusammenpasst.

Bei einem vierten Aspekt der vorliegenden Erfindung handelt es sich um die Verwendung einer erfindungsgemäßen Kappe zur Montage/Demontage einer Nadelanordnung an einer Injektionsvorrichtung.

Bei einem fünften Aspekt der vorliegenden Erfindung handelt es sich um die Verwendung einer erfindungsgemäßen Nadelanordnung in einer Injektionsvorrichtung mit einer erfindungsgemäßen Kappe.

Bei einem sechsten Aspekt der vorliegenden Erfindung handelt es sich um einen Satz mit einer erfindungsgemäßen Kappe, einer austauschbaren erfindungsgemäßen Nadelanordnung und/oder einer erfindungsgemäßen Injektionsvorrichtung.

### Beispiele

In den Figuren 1 bis 4 werden einige besondere Ausführungsformen der vorliegenden Erfindung als ein Beispiel gezeigt, ohne die Erfindung einzuschränken.
Figur 1 zeigt Seitenansichten von Kappen mit einer Klemme, wobei
   A eine Kappe mit einer Klemme, die wie ein Sechskant-Ringschlüssel ausgestaltet ist,
   B eine Kappe mit einer Klemme, die wie ein Schraubenschlüssel mit einem Gummiring ausgestaltet ist, die mit der Nadelanordnung form- und kraftschlüssig verbunden ist,
   C eine Kappe mit einer flexiblen Klemme, die wie ein Ringschlüssel ausgestaltet ist und
   D eine Kappe mit einer flexiblen Klemme, die wie ein Schraubenschlüssel mit einem Teilring aus Gummi ausgestaltet ist.
Figur 2 zeigt eine Draufsicht der Kappe, wobei die Vorderseite der Kappe die Aufnahmebohrung umfasst, die wie ein Steckschlüssel ausgestaltet ist und funktioniert.
In Figur 2A ist die Aufnahmebohrung sechseckig ausgestaltet, während die Aufnahmebohrung in Figur 2B wie ein Ringschlüssel ausgestaltet ist.
Figur 3 zeigt eine Seitenansicht der Kappe mit der Aufnahmebohrung auf der Vorderseite.
Figur 3A zeigt eine Seitenansicht einer form- und kraftschlüssigen Befestigung der Kappe, die als eine Aufnahmebohrung der Nadelanordnung dient.
Figur 3B zeigt eine Seitenansicht der Kappe, die als eine Aufnahmebohrung der Nadelanordnung dient.
Figur 4 zeigt eine Seitenansicht der Kappe mit der Aufnahmebohrung an einer Verlängerung am distalen Ende der Kappe.
Figur 4A zeigt eine Verlängerung mit einer Aufnahmebohrung entsprechend Figur 1A,
Figur 4B zeigt eine Verlängerung mit einer Aufnahmebohrung entsprechend Figur 1B,
Figur 4C zeigt eine Schnittansicht von Figur 4A, Figur 4D zeigt eine Schnittansicht von Figur 1B, Figur 4E zeigt eine Verlängerung mit einer Aufnahmebohrung gemäß einem Ringschlüssel.

## Patentansprüche

1. Injektionsvorrichtung zur transdermalen oder subkutanen Verabreichung von pharmazeutischen Zusammensetzungen ,wobei die Injektionsvorrichtung eine austauschbare Nadelanordnung und eine Kappe umfasst und wobei die Kappe eine Befestigung zur Befestigung der Kappe an der Vorrichtung aufweist, um die austauschbare Nadelanordnung abzudecken, wobei die Kappe eine Öffnung aufweist, wobei die Kappe als Montage-/Demontagewerkzeug dient und wobei die Kappe eine Aufnahmebohrung umfasst, wobei die Kappe mit der Aufnahmebohrung zur Aufnahme der Nadelanordnung ausgestaltet ist, wobei die Aufnahmebohrung wie ein Schraubenschlüssel oder Mutternschlüssel ausgestaltet ist, **dadurch gekennzeichnet, dass** der Schraubenschlüssel oder Mutternschlüssel auf die Größe des Durchmessers der Nadelanordnung einstellbar ist, wobei die Innenfläche der Aufnahmebohrung mit Gummi bedeckt ist.

2. Injektionsvorrichtung nach Anspruch 1,
wobei die Öffnung eine einseitige Öffnung ist.

3. Injektionsvorrichtung nach einem der Ansprüche 1 oder 2,
wobei die Aufnahmebohrung an einer Verlängerung am distalen Ende der Kappe ausgebildet ist.

4. Injektionsvorrichtung nach einem der Ansprüche 1 oder 2,
wobei die Kappe eine Klemme oder eine Befestigung umfasst und die Aufnahmebohrung durch die Klemme, die Befestigung oder durch eine Kombination der Kappe, der Klemme oder der Befestigung ausgebildet ist.

5. Injektionsvorrichtung nach einem der Ansprüche 1 bis 4,
wobei die Aufnahmebohrung als Sechskant- oder Mehrkantschlüssel ausgebildet ist.

6. Injektionsvorrichtung nach einem der Ansprüche 1 bis 5, aufweisend eine austauschbare Nadelanordnung mit der Geometrie solcher Bolzen, die mit der Aufnahmebohrung der Kappe zusammenpasst.

7. Injektionsvorrichtung nach einem der Ansprüche 1 bis 6,
wobei die Nadelanordnung eine Nadelkappe aufweist, die von der Kappe der Injektionsvorrichtung verschieden ist.

8. Verwendung einer Kappe einer Injektionsvorrichtung nach einem der vorhergehenden Ansprüche zur Montage/Demontage einer Nadelanordnung dieser Injektionsvorrichtung zur transdermalen oder subkutanen Verabreichung von pharmazeutischen Zusammensetzungen.

## Claims

1. Injection device for transdermal or subcutaneous administration of pharmaceutical compositions, wherein the injection device comprises an exchangeable needle assembly and a cap, and wherein the cap has an attachment for fixing the cap on the device in order to cover the exchangeable needle assembly, wherein the cap has an opening, wherein the cap serves as a mounting/ demounting tool, and wherein the cap comprises a receiving bore, wherein the cap is designed with the receiving bore to receive the needle assembly, wherein the receiving bore is designed as a spanner or wrench, **characterized in that** the spanner or wrench is adjustable to the size of the diameter of the needle assembly, wherein the inner face of the receiving bore is covered with rubber.

2. Injection device according to Claim 1, wherein the opening is an opening on one side.

3. Injection device according to either of Claims 1 and 2, wherein the receiving bore is formed on an elongation at the distal end of the cap.

4. Injection device according to either of Claims 1 and 2, wherein the cap comprises a clip or an attachment, and the receiving bore is formed by the clip, the attachment or by a combination of the cap, the clip or the attachment.

5. Injection device according to one of Claims 1 to 4, wherein the receiving bore is designed as a hexagonal or multi-edged spanner.

6. Injection device according to one of Claims 1 to 5, having an exchangeable needle assembly with the geometry of such bolts, which fits to the receiving bore of the cap.

7. Injection device according to one of Claims 1 to 6, wherein the needle assembly has a needle cap, which is different from the cap of the injection device.

8. Use of a cap of an injection device according to one of the preceding claims for the mounting/ demounting of a needle assembly of this injection device for transdermal or subcutaneous administration of pharmaceutical compositions.

## Revendications

1. Dispositif d'injection pour l'administration transdermique ou sous-cutanée de compositions pharmaceutiques, le dispositif d'injection comprenant un agencement d'aiguille remplaçable et un capuchon et le capuchon présentant une fixation pour fixer le capuchon au dispositif, de manière à recouvrir l'agencement d'aiguille remplaçable, le capuchon présentant une ouverture, le capuchon servant d'outil de montage/démontage et le capuchon comprenant un orifice de réception, le capuchon étant configuré avec l'orifice de réception de manière à recevoir l'agencement d'aiguille, l'orifice de réception étant configuré à la manière d'une clé à vis ou d'une clé à écrou, **caractérisé en ce que** la clé à vis ou la clé à écrou peut être ajustée à la taille du diamètre de l'agencement d'aiguille, la surface intérieure de l'orifice de réception étant recouverte de caoutchouc.

2. Dispositif d'injection selon la revendication 1, dans lequel l'ouverture est une ouverture d'un côté.

3. Dispositif d'injection selon l'une quelconque des revendications 1 et 2, dans lequel l'orifice de réception est réalisé au niveau d'un prolongement à l'extrémité distale du capuchon.

4. Dispositif d'injection selon l'une quelconque des revendications 1 et 2, dans lequel le capuchon comprend une pince ou une fixation et l'orifice de réception est réalisé par la pince, la fixation ou par une combinaison du capuchon, de la pince ou de la fixation.

5. Dispositif d'injection selon l'une quelconque des revendications 1 à 4, dans lequel l'orifice de réception est réalisé sous forme de clé hexagonale ou de clé polygonale.

6. Dispositif d'injection selon l'une quelconque des revendications 1 à 5, présentant un agencement d'aiguille remplaçable ayant une géométrie de boulons qui est adaptée à l'orifice de réception du capuchon.

7. Dispositif d'injection selon l'une quelconque des revendications 1 à 6, dans lequel l'agencement d'aiguille présente un capuchon d'aiguille qui est différent du capuchon du dispositif d'injection.

8. Utilisation d'un capuchon d'un dispositif d'injection selon l'une quelconque des revendications précédentes pour le montage/démontage d'un agencement d'aiguille de ce dispositif d'injection pour l'administration transdermique ou sous-cutanée de compositions pharmaceutiques.
